(19)

Europäisches
Patentamt
European
Patent Office
Office européen
des brevets

(11) **EP 3 318 176 A1**

(12) **EUROPEAN PATENT APPLICATION**
published in accordance with Art. 153(4) EPC

(43) Date of publication:
**09.05.2018 Bulletin 2018/19**

(21) Application number: **16821045.8**

(22) Date of filing: **16.02.2016**

(51) Int Cl.:
*A61B 1/04* (2006.01)   *A61B 1/00* (2006.01)
*G02B 23/24* (2006.01)   *H04N 7/18* (2006.01)

(86) International application number:
**PCT/JP2016/054452**

(87) International publication number:
**WO 2017/006574 (12.01.2017 Gazette 2017/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **03.07.2015 JP 2015134725**

(71) Applicant: **OLYMPUS CORPORATION**
**Hachioji-shi**
**Tokyo 192-8507 (JP)**

(72) Inventor: **SATO, Daisuke**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **IMAGE PROCESSING DEVICE, IMAGE DETERMINATION SYSTEM, AND ENDOSCOPE SYSTEM**

(57) Provided is an image processing apparatus or the like capable of accurately performing discrimination on whether or not an area where a subject is in a specific state is included in an image with respect to images acquired by capturing the inside of a living body, with simple arithmetic processing. An image processing apparatus that performs image processing based on image data representing an image of a subject output from an imaging unit and distance measurement data representing a distance to the subject, the image processing apparatus including: a depth image creation unit 337 that calculates a depth from the imaging unit to the subject based on the distance measurement data; a subject distance calculation unit 336 that calculates a subject distance between the imaging unit and the subject based on the image data; a difference calculation unit 338 that calculates a difference between the depth calculated by the depth image creation unit 337 and the subject distance calculated by the subject distance calculation unit 336; and a discrimination unit 339 that discriminates whether or not an area where a surface of the subject is in a specific state is included in an image in which the subject is imaged based on the difference calculated by the difference calculation unit 338.

FIG.3

**Description**

Field

The present invention relates to an image processing apparatus, an image discrimination system, and an endoscopic system that perform image processing based on data acquired by capturing an image in a living body.

Background

A technique, which extracts an image on which an area where a subject is in a specific state, such as an abnormal portion of a lesion or the like is imaged or identifying a type of abnormality with respect to an image group acquired by capturing the inside of a living body using a medical observation apparatus such as an endoscope and a capsule endoscope, has been known.

When a lot of images are acquired by a one-time examination using a medical observation apparatus, it is a heavy burden for a doctor to observe all the images. In addition, when the type of abnormality is automatically identified by image processing, a load to an image processing apparatus is extremely high if all the images are set as processing targets. Thus, a technique of performing discrimination, with respect to each image acquired by an examination, on whether or not each image is an image that needs to be observed in detail by a doctor or whether or not each image is an image that needs to be subjected to abnormality identification processing, as preprocessing before the doctor performs detailed observation or performs automatic identification processing of a type of abnormality is extremely useful.

For example, Patent Literature 1 discloses a technique of modeling gradient variations of pixel values in an intraluminal image, and detecting an abnormality candidate area from the intraluminal image according to a difference between a pixel value of each pixel constituting the intraluminal image and an estimated pixel value of each pixel, the estimated pixel value being determined according to the modeled gradient variations of the pixel values.

Citation List

Patent Literature

Patent Literature 1: JP 2009-297450 A

Summary

Technical Problem

In the case of the above-described Patent Literature 1, it is necessary to prepare the gradient variations of pixel values, in advance, by modeling, and arithmetic processing thereof is complicated. However, it is desirable to perform processing which enables accurate discrimination with arithmetic processing as simple as possible, as preprocessing for narrowing down the image that needs to be observed in detail by the doctor or the image that needs to be subjected to the automatic identification processing.

The present invention has been made in view of the above description, and an object thereof is to provide an image processing apparatus, an image discrimination system, and an endoscopic system capable of accurately performing discrimination on whether or not an area where a subject is in a specific state is included in an image with respect to images acquired by capturing the inside of a living body, with simple arithmetic processing.

Solution to Problem

To solve the above-described problem and achieve the object, an image processing apparatus according to the present invention performs image processing based on image data and distance measurement data output from an imaging unit including an illumination unit configured to generate illumination light for irradiation of a subject, a condensing optical system configured to condense the illumination light reflected by the subject, and an image sensor configured to receive the illumination light condensed by the condensing optical system and to output the image data representing an image of the subject and output the distance measurement data representing a distance to the subject based on the illumination light, and includes: a depth calculation unit configured to calculate a depth from the imaging unit to the subject based on the distance measurement data; a subject distance calculation unit configured to calculate a subject distance between the imaging unit and the subject based on the image data; a difference calculation unit configured to calculate a difference between the depth calculated by the depth calculation unit and the subject distance calculated by the subject distance calculation unit; and a discrimination unit configured to discriminate whether or not an area where a surface of the subject is in a specific state is included in an image in which the subject is imaged based on the difference calculated by the difference calculation unit.

In the above-described image processing apparatus, the depth calculation unit calculates depths to a plurality of points on the subject imaged on a plurality of pixels forming the image, the subject distance calculation unit calculates at least subject distances to a plurality of points on the subject for which the depth calculation unit calculates the depth, among the plurality of pixels forming the image, the difference calculation unit calculates difference values between distances to a common point, between the plurality of depths calculated by the depth calculation unit and the plurality of subject distances calculated by the subject distance calculation unit, and the discrimination unit performs discrimination based on a statistical value of the plurality of difference values calculated by the difference calculation unit.

In the above-described image processing apparatus, the discrimination unit performs the discrimination by comparing a difference value having a peak frequency among the plurality of difference values with a threshold value.

The above-described image processing apparatus further includes an identification unit configured to perform identification processing to identify a type of the specific state with respect to an image that is discriminated to include the area in the specific state by the discrimination unit.

In the above-described image processing apparatus, the subject is a mucosa of a living body, and the specific state is an abnormal state in which the mucosa is changed from a normal state.

An image discrimination system according to the present invention includes: the image processing apparatus; and the imaging unit.

In the above-described image discrimination system, the image sensor includes: a plurality of imaging pixels that receives the illumination light and outputs an electric signal representing the image of the subject; a plurality of distance measurement pixels that receives the illumination light and outputs an electric signal containing information on the distance to the subject; and a signal processing circuit that outputs the image data based on the electrical signal output from each of the plurality of imaging pixels and outputs the distance measurement data based on the electric signal output from each of the plurality of distance measurement pixels.

An endoscopic system according to the present invention includes: the image processing apparatus; and a capsule endoscope accommodating the imaging unit in a capsule-shaped casing.

An endoscopic system according to the present invention includes: the image processing apparatus; and a video scope provided with the imaging unit at a distal end portion of an insertion portion to be inserted into the subject.

Advantageous Effects of Invention

According to the present invention, a subject distance is calculated based on image data, which depends on a reflection characteristic of a subject surface, a depth to the subject is calculated based on distance measurement data which does not depend on the reflection characteristic of the subject surface, and a difference between the subject distance and the subject is calculated, and thus, it is possible to numerically grasp a change in the reflection characteristic on the subject surface. Therefore, it is possible to accurately perform discrimination on whether or not the area where the subject is in the specific state is included in the image with respect to the images acquired by capturing the inside of the living body, with the simple arithmetic processing.

Brief Description of Drawings

FIG. 1 is a schematic diagram illustrating a configuration example of an image discrimination system according to a first embodiment of the present invention.

FIG. 2 is a schematic diagram illustrating a light receiving surface of an image sensor illustrated in FIG. 1.

FIG. 3 is a block diagram illustrating a configuration of an image discrimination unit illustrated in FIG. 1.

FIG. 4 is a schematic diagram for describing a measurement principle of a subject distance.

FIG. 5 is a schematic diagram illustrating a subject distance image and a depth image in a case where the entire image is a normal mucosal area.

FIG. 6 is a histogram of difference values between the subject distance image and the depth image illustrated in FIG. 5.

FIG. 7 is a schematic diagram illustrating a subject distance image and a depth image in a case where the entire image is an abnormal area.

FIG. 8 is a histogram of difference values between the subject distance image and the depth image illustrated in FIG. 7.

FIG. 9 is a schematic diagram illustrating a subject distance image and a depth image in a case where an abnormal area is included in a part of an image.

FIG. 10 is a histogram of difference values between the subject distance image and the depth image illustrated in FIG. 9.

FIG. 11 is a schematic diagram illustrating a configuration example of an image discrimination system according to a second embodiment of the present invention.

FIG. 12 is a schematic diagram for describing an operation of an arithmetic unit illustrated in FIG. 11.

FIG. 13 is a schematic diagram illustrating a configuration example of an endoscopic system according to a third embodiment of the present invention.

FIG. 14 is a schematic diagram illustrating an example of an internal structure of a capsule endoscope illustrated in FIG. 13.

FIG. 15 is a schematic diagram illustrating a configuration example of an endoscopic system according to a fourth embodiment of the present invention.

Description of Embodiments

Hereinafter, an image processing apparatus, an image discrimination system, and an endoscopic system according to embodiments of the present invention will be described with reference to the drawings. In the following description, the respective drawings schematically illustrate shapes, sizes, and positional relationships merely to such a degree that the content of the present invention is understandable. Accordingly, the present invention is not limited only to the shapes, sizes, and positional relationships exemplified in the respective drawings. Incidentally, the same parts are denoted by the same reference signs in the description of the drawings.

(First Embodiment)

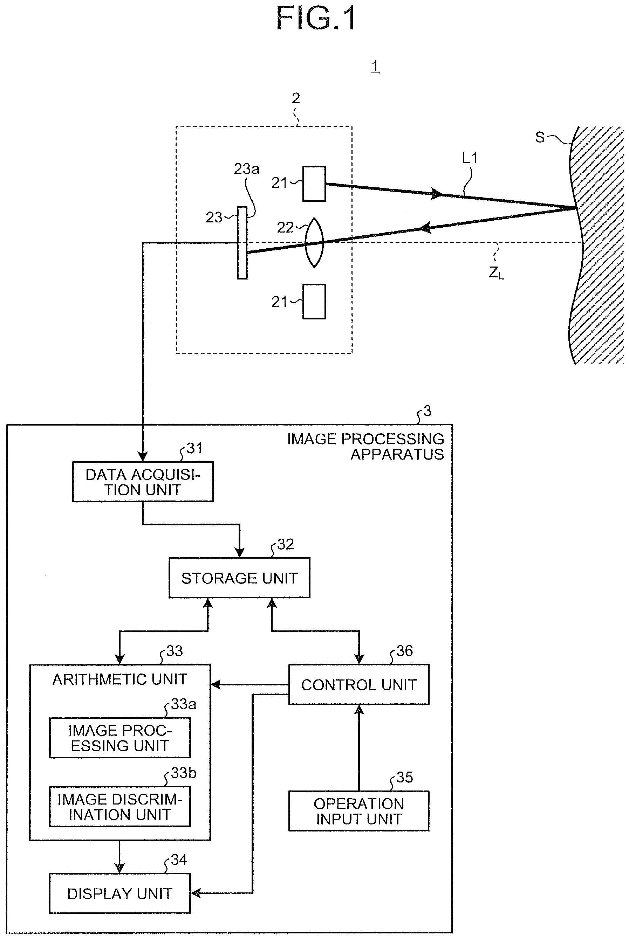

FIG. 1 is a schematic diagram illustrating a configuration example of an image discrimination system according to a first embodiment of the present invention. An image discrimination system 1 according to the first embodiment is a system which is applied to an endoscopic system or the like that is introduced into a living body to perform imaging, and discriminates whether or not an area where a subject is in a specific state (hereinafter, also referred to as a specific area) is included in an image acquired by capturing the subject such as a mucosa. The endoscopic system may be a general endoscopic system which includes a video scope provided with an imaging unit at a distal end portion of an insertion portion, or a capsule endoscopic system that introduces a capsule endoscope incorporating an imaging unit and a wireless communication unit into a living body and executes imaging.

Here, the specific area is an area (abnormal area) where the mucosa of the living body as the subject is changed from a normal state to an abnormal state, and specifically, includes a lesion area where lesions such as bleeding, a tumor, and an ulcer occur, a candidate area where the possibility of lesions is estimated, and the like. Alternatively, an area where a subject other than the mucosa is imaged, such as a residue area where a residue is imaged, a bubble area where a bubble is imaged, and a treatment tool area where a treatment tool used for treatment of the living body, such as a clip, is imaged, may be discriminated as the specific area. In addition, it may be configured such that a normal mucosal area is set as the specific area and whether or not an image as a processing target is an image including only the normal mucosal area is discriminated.

As illustrated in FIG. 1, the image discrimination system 1 includes an imaging unit 2 which generates and outputs image data by capturing a subject S and generates and outputs distance measurement data by actually measuring a distance to the subject S, and an image processing apparatus 3 which acquires the image data and the distance measurement data output from the imaging unit 2, creates an image of the subject S based on the image data, and discriminates whether or not the specific area is included in the image based on the image data and the distance measurement data.

The imaging unit 2 includes one or more illumination units 21 that generate illumination light to illuminate the subject S, a condensing optical system 22 such as a condenser lens, and an image sensor 23.

The illumination unit 21 includes a light emitting element such as a light emitting diode (LED) and a driving circuit to drive the light emitting element, and generates white light or illumination light of a specific frequency band and irradiates the subject S with the generated light.

The image sensor 23 is a sensor capable of acquiring the image data representing visual information of the subject S and the distance measurement data representing a depth to the subject S, and has a light receiving surface 23a to receive the illumination light (that is, reflection light) emitted from the illumination unit 21, reflected by the subject S, and condensed by the condensing optical system 22. In the first embodiment, a sensor for image plane phase difference AF is used as the image sensor 23.

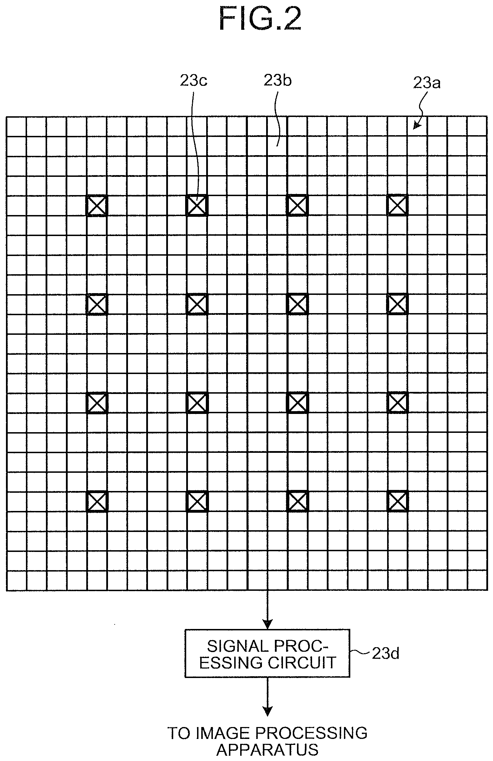

FIG. 2 is a schematic diagram for describing a configuration of the image sensor 23. As illustrated in FIG. 2, the image sensor 23 includes a plurality of imaging pixels 23b and distance measurement pixels 23c arranged on the light receiving surface 23a, and a signal processing circuit 23d which processes electric signals output from these pixels. The plurality of imaging pixels 23b is arranged in a matrix on the light receiving surface 23a, and the plurality of distance measurement pixels 23c is arranged so as to replace a part of this matrix. In FIG. 2, a mark "x" is attached at the position of the distance measurement pixel 23c to distinguish the distance measurement pixel 23c from the imaging pixel 23b.

Each of the imaging pixels 23b has a structure in which a microlens and a color filter of any one of red (R), green (G), and blue (B) are stacked on a photoelectric converter such as a photodiode to generate a charge corresponding to the light amount of light incident on the photoelectric converter. The imaging pixels 23b are arranged in a predetermined arrangement order such as a Bayer arrangement depending on each color of the color filter included in each pixel. The signal processing circuit 23d converts the charge generated by the respective imaging pixels 23b into a voltage signal, further converts the voltage signal into a digital signal, and outputs the digital signal as the image data.

Each of the distance measurement pixels 23c has a structure in which two photoelectric converters are arranged side by side on the same plane and one microlens is arranged so as to straddle over these photoelectric converters. The light incident on the microlens is incident on the two photoelectric converters with distribution according to an incident position on the microlens. Each of the two photoelectric converters generates a charge corresponding to the light amount of the incident light. The signal processing circuit 23d converts the charge generated in each of the two photoelectric converters of each of the distance measurement pixels 23c into a voltage signal, and generates and outputs the distance measurement data representing the distance (depth) from the imaging unit 2 to the subject S based on a phase difference (information on the distance) between these voltage signals.

The image processing apparatus 3 includes a data acquisition unit 31 which acquires the image data and the distance measurement data output from the imaging unit 2, a storage unit 32 which stores the image data and the distance measurement data acquired by the data acquisition unit 31 and various programs and parameters used in the image processing apparatus 3, an arithmetic unit 33 which performs various types of arithmetic processing based on the image data and the distance measurement data, a display unit 34 which displays the image of the subject S and the like, an operation input unit 35 which is used for input of various types of information and commands with respect to the image processing apparatus 3, and a control unit 36 which comprehensively controls these respective units.

The data acquisition unit 31 is appropriately configured according to an aspect of the endoscopic system to which the image discrimination system 1 is applied. For example, in the case of the general endoscopic system in which the video scope is inserted into a body, the data acquisition unit 31 is configured using an interface that takes the image data and the distance measurement data generated by the imaging unit 2 provided in the video scope. In addition, in the case of the capsule endoscopic system, the data acquisition unit 31 is configured using a reception unit that receives a signal wirelessly transmitted from the capsule endoscope via an antenna. Alternatively, the image data and the distance measurement data may be exchanged using a portable storage medium with the capsule endoscope, and in this case, the data acquisition unit 31 is configured using a reader device to which the portable storage medium is detachably attached and which reads the stored image data and distance measurement data. Alternatively, when a server to store the image data and the distance measurement data generated in the endoscopic system is installed, the data acquisition unit 31 is configured using a communication device or the like connected to the server and acquires various types of data by performing data communication with the server.

The storage unit 32 is configured of an information storage device such as various integrated circuit (IC) memories, such as a read only memory (ROM) and a random access memory (RAM), for example, an update recordable flash memory or the like, and a hard disk or a compact disc read only memory (CD-ROM), which is built-in or connected using a data communication terminal, and a device for writing and reading information to and from the information storage device. The storage unit 32 stores a program, configured to operate the image processing apparatus 3 and cause the image processing apparatus 3 to execute various functions, data used during execution of this program, the image data and the distance measurement data acquired by the data acquisition unit 31, for example, various parameters, and the like.

Specific examples of the parameters stored in the storage unit 32 may include a reflection characteristic of the living body as the subject S used for operation executed by the arithmetic unit 33, that is, a reflectance of a mucosal surface. The storage unit 32 may store a plurality of reflectances corresponding to types of subjects as observation targets, such as a stomach mucosa and a large intestine mucosa, as the parameters.

The arithmetic unit 33 is configured using a general-purpose processor, such as a central processing unit (CPU) or a dedicated processor such as various arithmetic circuits to execute specific functions, for example, an application specific integrated circuit (ASIC) and the like. When the arithmetic unit 33 is the general-purpose processor, the arithmetic processing is executed by reading various operation programs stored in the storage unit 32. In addition, when the arithmetic unit 33 is the dedicated processor, the processor may independently execute various types of arithmetic processing, or the processor and the storage unit 32 may execute the arithmetic processing in collaboration or combination with each other using various types of data stored in the storage unit 32.

To be specific, the arithmetic unit 33 includes an image processing unit 33a which generates an image for display by executing predetermined image processing, such as white balance processing, demosaicing, gamma conversion, and smoothing (noise removal and the like), with respect to the image data, and an image discrimination unit 33b which discriminates whether or not the specific area, such as the abnormal area, is included in the image generated by the image processing unit 33a based on the image data and the distance measurement data. Detailed configuration and operation of the image discrimination unit 33b will be described later.

The display unit 34 includes various displays, such as a liquid crystal display and an organic electro luminescence (EL) display, and displays information such as the image generated by the image processing unit 33a and a distance calculated by the image discrimination unit 33b.

The control unit 36 is configured using a general-purpose processor such as a CPU or a dedicated processor such as various arithmetic circuits to execute specific functions, for example, an ASIC and the like. When the control unit 36 is the general-purpose processor, a control program stored in the storage unit 32 is read to perform transfer of a command and data to the respective units forming the image processing apparatus 3, and the overall operation of the image processing apparatus 3 is comprehensively controlled. In addition, when the control unit 36 is the dedicated processor, the processor may independently execute various types of processing, or the processor and the storage unit 32 may execute various types of processing in collaboration or combination with each other using various types of data stored in the storage unit 32.

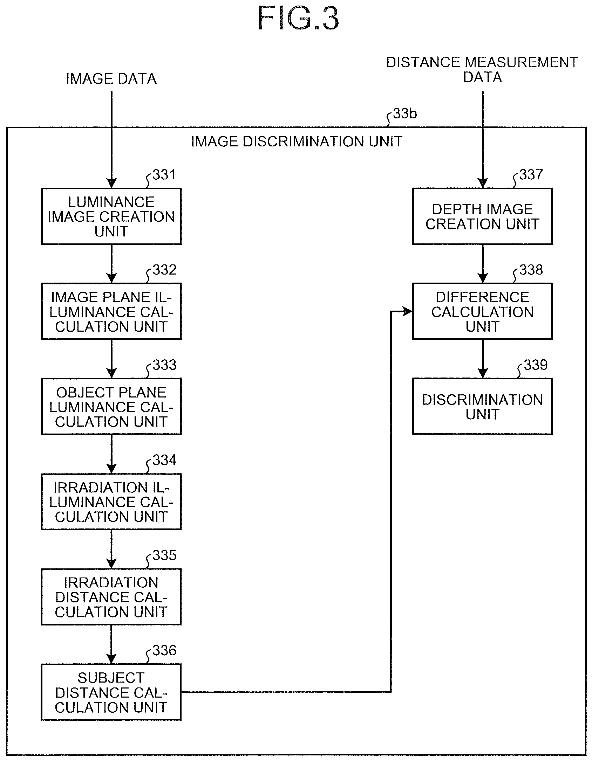

FIG. 3 is a block diagram illustrating the detailed configuration of the image discrimination unit 33b. As illustrated in FIG. 3, the image discrimination unit 33b includes a luminance image creation unit 331, an image plane illuminance calculation unit 332, an object plane luminance calculation unit 333, an irradiation illuminance calculation unit 334, an irradiation distance calculation unit 335, a subject distance calculation unit 336, a depth image creation unit (depth calculation unit) 337, a difference calculation unit 338, and a discrimination unit 339.

The luminance image creation unit 331 creates a luminance image in which luminance of the image of the subject S is a pixel value of each pixel based on the image data read from the storage unit 32.

The image plane illuminance calculation unit 332 calculates illuminance on an image plane of the image sensor 23 based on the luminance image created by the luminance image creation unit 331.

The object plane luminance calculation unit 333 calculates luminance on a surface of the subject S based on the illuminance on the image plane calculated by the image plane illuminance calculation unit 332.

The irradiation illuminance calculation unit 334 calculates irradiation illuminance of illumination light with which the subject S is irradiated based on the luminance on the object plane calculated by the object plane luminance calculation unit 333.

The irradiation distance calculation unit 335 calculates an irradiation distance from the condensing optical system 22 to the subject S based on the irradiation illuminance of the illumination light calculated by the irradiation illuminance calculation unit 334.

The subject distance calculation unit 336 calculates a subject distance, which is a distance obtained by projecting the irradiation distance calculated by the irradiation distance calculation unit 335 onto an optical axis $Z_L$ of the condensing optical system 22, and creates a subject distance image in which the calculated subject distance is a pixel value of each pixel.

The depth image creation unit 337 creates a depth image in which a depth between the point on the subject S, which corresponds to each pixel position inside the image created by the image processing unit 33a, and the condensing optical system 22 is a pixel value of each pixel based on the distance measurement data read from the storage unit 32 Incidentally, the distance measurement pixels 23c are sparsely arranged on the light receiving surface 23a as described above. For a pixel position where the distance measurement pixel 23c is not arranged, the depth image creation unit 337 may set a null value or calculate a depth by interpolation operation using the distance measurement data output from the distance measurement pixel 23c arranged in the vicinity thereof.

The difference calculation unit 338 calculates a difference between the subject distance image created by the subject distance calculation unit 336 and the depth image created by the depth image creation unit 337. That is, a difference value between the subject distance and the depth of pixels which are common between both the images is obtained.

The discrimination unit 339 discriminates whether or not the specific area is included in the image where the subject S is imaged based on a statistical value of the difference value calculated by the difference calculation unit 338.

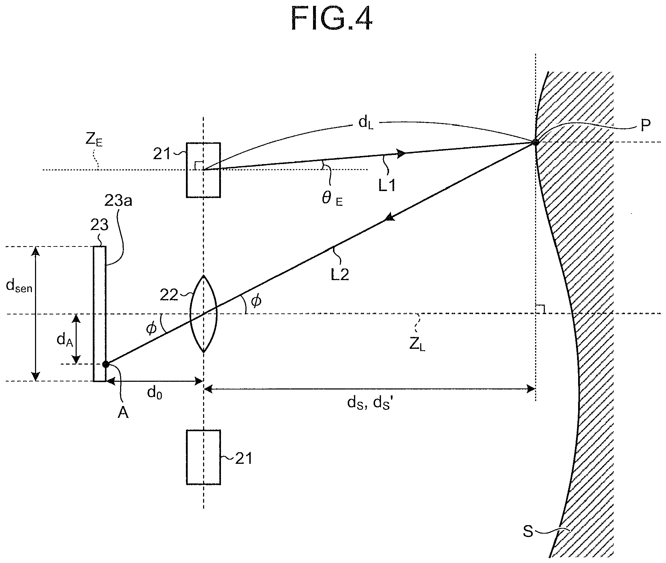

Next, a method of discriminating an image according to the first embodiment will be described in detail with reference to FIGS. 1 to 10. FIG. 4 is a schematic diagram illustrating positional and angular relationships between each unit in the imaging unit 2 and the subject S.

First, the image discrimination system 1 causes the illumination unit 21 to generate light to irradiate the subject S with illumination light L1. Accordingly, the illumination light (reflection light) reflected by the subject S is condensed by the condensing optical system 22 and is incident on the light receiving surface 23a of the image sensor 23. The image sensor 23 outputs the image data at each position of the imaging pixels 23b and outputs the distance measurement data at each position of the distance measurement pixels 23c based on output signals from the imaging pixels 23b and the distance measurement pixels 23c (see FIG. 2) arranged on the light receiving surface 23a. The data acquisition unit 31 of the image processing apparatus 3 takes these image data and distance measurement data and stores these data in the storage unit 32.

As illustrated in FIG. 3, the image discrimination unit 33b takes in the image data and the distance measurement data from the storage unit 32, inputs the image data to the luminance image creation unit 331, and inputs the distance measurement data to the depth image creation unit 337.

The luminance image creation unit 331 creates the luminance image in which the luminance of the image of the subject S is the pixel value based on the input image data. Here, since the distance measurement pixels 23c are sparsely arranged on the light receiving surface 23a of the image sensor 23 as illustrated in FIG. 2, the image data is not acquired at a pixel position where the distance measurement pixel 23c is arranged. Thus, the luminance image creation unit 331 calculates luminance at the position of the distance measurement pixel 23c by interpolation using the image data based on an output value from the imaging pixel 23b positioned in the vicinity of the distance measurement pixel 23c.

Subsequently, the image plane illuminance calculation unit 332 calculates illuminance (image plane illuminance) $E_f$ [lx] at a pixel of interest A on the light receiving surface 23a, which is the image plane of the condensing optical system 22, based on the luminance image created by the luminance image creation unit 331. Here, the image plane illuminance is illuminance when reflection light L2 having passed through the condensing optical system 22 is incident on the image sensor 23 at the time of regarding the condensing optical system 22 as an illumination system.

The image plane illuminance $E_f$ is given by the following Formula (1) using an output value $V_{out}$ from the imaging pixel 23b (see FIG. 2) at the position of the pixel of interest A on the light receiving surface 23a, a coefficient K, and an exposure time t. The coefficient K is a total coefficient in consideration of an absorption coefficient of light in each of the imaging pixels 23b, a coefficient of conversion from a charge to a voltage, a gain or a loss in a circuit such as AD converter and an amplifier, and the like, and is set in advance according to the specification of the image sensor 23. Incidentally, the image plane illuminance $E_f$ at the position of each of the distance measurement pixels 23c is calculated by interpolation using the output value $V_{out}$ from the imaging pixel 23b in the vicinity of the corresponding distance measurement pixel 23c.

$$E_f = V_{out} \times \frac{1}{K} \times \frac{1}{t} \qquad (1)$$

Subsequently, the object plane luminance calculation unit 333 calculates object plane luminance $L_S$ [cd/m²], which is the luminance on the surface of the subject S, based on the image plane illuminance $E_f$. The object plane luminance $L_S$ is given by the following Formula (2) using the image plane illuminance $E_f$, an aperture diameter D of the condensing optical system 22, a focal length b, and an intensity transmittance T(h).

$$L_s = E_f \times \frac{4}{\pi} \times \frac{b^2}{D^2} \times \frac{1}{T(h)} \qquad (2)$$

Subsequently, the irradiation illuminance calculation unit 334 calculates irradiation illuminance $E_0$ [lx] of the illumination light L1 with which the subject S is irradiated based on the object plane luminance $L_S$. The illumination light L1 is attenuated by a reflectance $R_0$ on the surface of the subject S by being reflected at a point of interest P of the subject S. Accordingly, the irradiation illuminance $E_0$ can be inversely calculated by the following Formula (3) using the object plane luminance $L_S$ and the reflectance $R_0$ of the subject S.

$$E_0 = L_s \times \frac{\pi}{R_0} \qquad (3)$$

Here, the reflectance $R_0$ is a value determined according to a surface property of the subject S, and is stored in advance in the storage unit 32. When the storage unit 32 stores the plurality of reflectances $R_0$ according to the type of the subject, the irradiation illuminance calculation unit 334 selects and uses the reflection $R_0$ according to the signal input from the operation input unit 35 (see FIG. 1).

The irradiation illuminance $E_0$ calculated in this manner is generated when the illumination light L1 emitted from the illumination unit 21 reaches the point of interest P of the subject S. During this time, the illumination light L1 emitted from the illumination unit 21 is attenuated by an irradiation distance $d_L$ to the point of interest P. Accordingly, a relationship of the following Formula (4) is established between luminance $L_{LED}$ of the illumination unit 21 and the irradiation illuminance $E_0$ at the point of interest P.

$$E_0 = \frac{4 \cdot L_{LED} \cdot S_{LED} \cdot Em_{SPE}}{d_L^2} \qquad (4)$$

In Formula (4), reference sign $S_{LED}$ indicates the surface area of an area where the illumination light L1 is emitted from the illumination unit 21. In addition, reference sign $Em_{SPE}$ is a spectral characteristic coefficient of the illumination light L1.

Accordingly, an irradiation distance $d_L$ [m] is given by the following Formula (5).

$$d_L = \sqrt{\frac{4 \cdot L_{LED} \cdot S_{LED} \cdot Em_{SPE}}{E_0}} \qquad (5)$$

Subsequently, the subject distance calculation unit 336 calculates a subject distance $d_S$ by projecting the irradiation distance $d_L$ on the optical axis $Z_L$ by the following Formula (6) using an angle of view $\phi$.

$$d_S = d_L \cdot \cos\phi \qquad (6)$$

Here, strictly speaking, the subject distance $d_S$ is $\cos\theta_E$ of the irradiation distance $d_L$ when an angle formed by a radiation direction of the illumination light L1 and an optical axis $Z_E$ of the illumination unit 21 is set as a radiation angle $\theta_E$. However, when the distance between the illumination unit 21 and the condensing optical system 22 of the imaging unit 2 is shorter than the subject distance $d_S$, the radiation angle $\theta_E$ can be approximated to the angle of view $\phi$.

The angle of view $\phi$ is calculated as follows. The pixel A (see FIG. 4) on the light receiving surface 23a corresponding to the pixel of interest in the luminance image created by the luminance image creation unit 331 is extracted, and a coordinate value of the pixel A is converted from the pixel into a distance (mm) using the number of pixels (pixels) of the image sensor 23 and the sensor size $d_{sen}$ (mm). Further, a distance from the optical axis $Z_L$ of the condensing optical system 22 to the pixel A, that is, an image height $d_A$ is calculated using the coordinate value of the pixel A converted into the distance. Further, the angle of view $\phi$ is calculated from a distance (design value) $d_0$ between the condensing optical system 22 and the light receiving surface 23a and the image height $d_A$ by the following Formula (7).

$$\phi = \tan^{-1}(d_A/d_0) \qquad (7)$$

The image plane illuminance calculation unit 332 to the subject distance calculation unit 336 perform such calculation processing of the subject distance $d_s$ for each pixel on the light receiving surface 23a, and creates a subject distance image having the subject distance $d_s$ as the pixel value of each pixel.

On the other hand, the depth image creation unit 337 creates a depth image of a size corresponding to the entire light receiving surface 23a in which a depth $d_s$' (see FIG. 4) from the condensing optical system 22 to the subject S is the pixel value of each pixel, based on the input distance measurement data. Here, the distance measurement pixels 23c are only sparsely arranged on the light receiving surface 23a of the image sensor 23 as illustrated in FIG. 2. Thus, the depth image creation unit 337 determines the pixel value using the distance measurement data based on the output value from the distance measurement pixel 23c for the pixel in the depth image corresponding to the position of the distance measurement pixel 23c. In addition, pixel values of the other pixels may be set as null values or may be determined by interpolation calculation using values of peripheral pixels.

The difference calculation unit 338 acquires the subject distance image created by the subject distance calculation unit 336 and the depth image created by the depth image creation unit 337, and calculates pixel values between pixels whose positions correspond to each other between these images, that is, a difference value between the subject distance $d_s$ and the depth $d_s$'. At this time, it is unnecessary to perform the difference calculation for the pixel whose pixel value is the null value in the depth image.

The discrimination unit 339 discriminates whether or not the specific area, such as the abnormal area, is included in the image created based on the image data input to the image discrimination unit 33b based on a frequency of the difference value calculated by the difference calculation unit 338. To be specific, a histogram of difference values is created, and the above-described discrimination is performed by comparing a mode of the difference value with a threshold value. Hereinafter, this discrimination method will be described in detail.

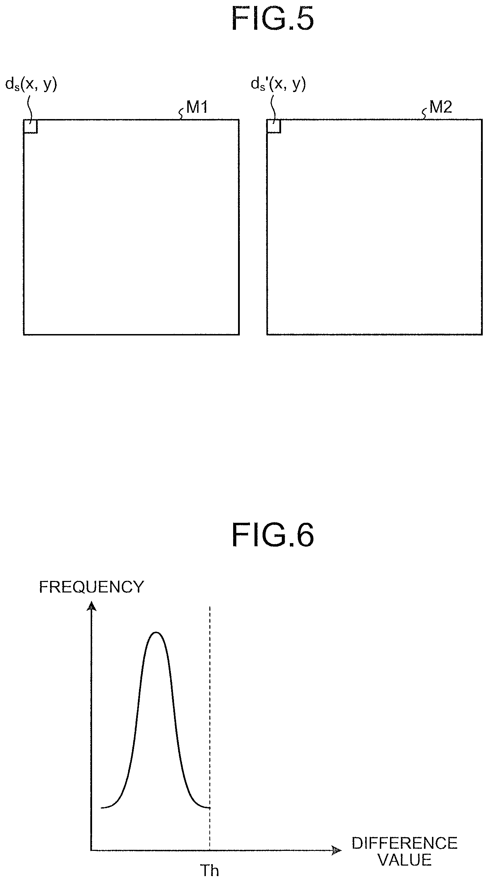

FIG. 5 is a schematic diagram illustrating the subject distance image and the depth image in a case where the entire image is the normal mucosal area. In addition, FIG. 6 is a histogram of the difference value between the subject distance image and the depth image illustrated in FIG. 5.

In this case, a difference value between a subject distance $d_s(x,y)$ at a position of each pixel forming a subject distance image M1 and a depth $d_s$' (x,y) at a position of each pixel forming a depth image M2 is substantially uniform as illustrated in FIG. 6. This indicates that the reflectance on the surface of the subject S affecting a calculation result of the subject distance $d_s(x,y)$ is substantially uniform throughout the image.

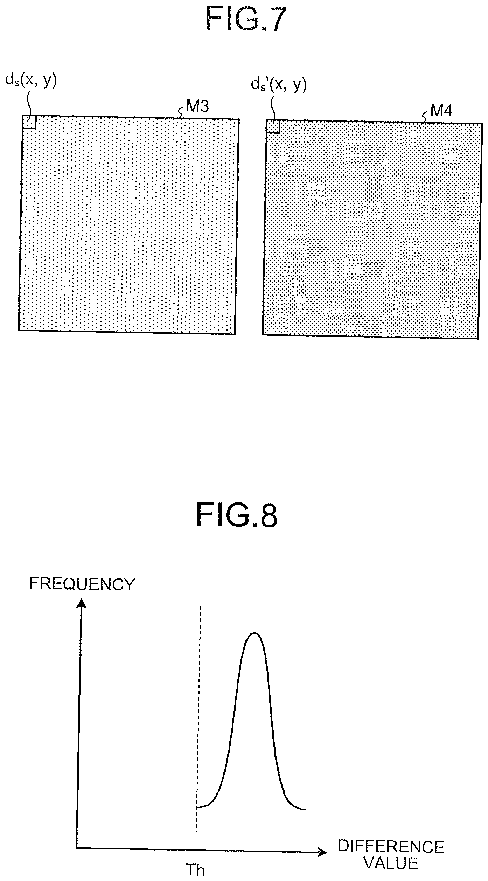

FIG. 7 is a schematic diagram illustrating the subject distance image and the depth image in a case where the entire image is the abnormal area. In addition, FIG. 8 is a histogram of the difference value between the subject distance image and the depth image illustrated in FIG. 7.

In this case, a difference value between a subject distance $d_s(x,y)$ at a position of each pixel forming a subject distance image M3 and a depth $d_s$' $(x,y)$ at a position of each pixel forming a depth image M4 is substantially uniform as illustrated in FIG. 8. This indicates that the reflectance on the surface of the subject S is substantially uniform. However, a difference value (mode) at the peak of the histogram is a value different from that in the case of the normal mucosal area (see FIG. 6). This is because the reflectance varies depending on whether a mucosa on the surface of the subject S is normal or abnormal.

Thus, it is possible to discriminate whether the image is the normal mucosal area or the abnormal area by comparing the difference value, which is the peak in the histogram of the difference values between the subject distance $d_s(x,y)$ and the depth $d_s$' $(x,y)$, with a threshold value Th. For example, it is possible to discriminate as the normal mucosal area when the difference value at the peak is smaller than the threshold value Th or as the abnormal area when the difference value at the peak is equal to or larger than the threshold value Th in the case of FIGS. 6 and 8.

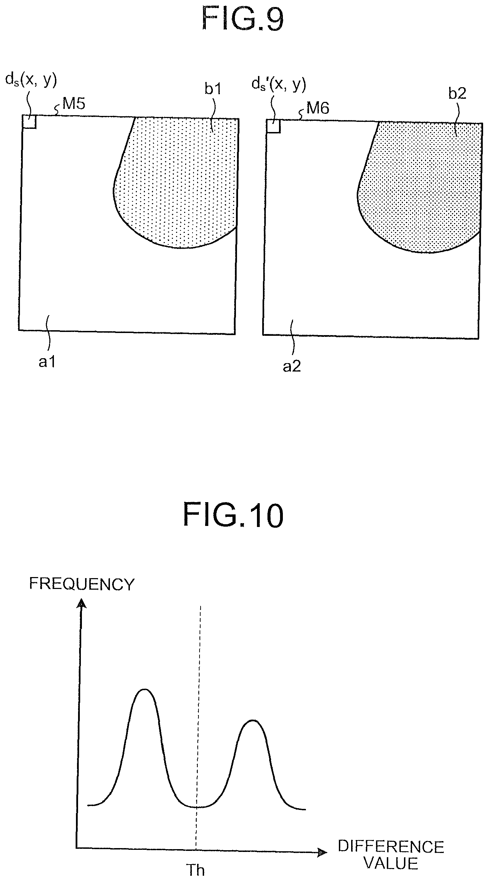

FIG. 9 is a schematic diagram illustrating the subject distance image and the depth image in a case where the abnormal area is included in a part of the image. In addition, FIG. 10 is a histogram of the difference values between the subject distance image and the depth image illustrated in FIG. 9.

In this case, a difference value between a subject distance $d_s(x,y)$ at a position of each pixel forming a subject distance image M5 and a depth $d_s$' $(x,y)$ at a position of each pixel forming a depth image M6 greatly varies as illustrated in FIG. 10. This indicates that the reflectance on the surface of the subject S affecting a calculation result of the subject distance $d_s(x,y)$ is not uniform. That is, this means that there are a plurality of parts having different reflectances in the subject S.

In detail, a peak of the difference value appears at a position close to the peak illustrated in FIG. 6 between a normal mucosal area a1 in the subject distance image M5 and a normal mucosal area a2 in the depth image M6. On the other hand, a peak of the difference value appears at a position close to the peak in FIG. 8 between an abnormal area b1 in the subject distance image M5 and an abnormal area b2 in the depth image M6.

The discrimination unit 339 compares the difference value at the peak of the histogram with the threshold value in this manner, thereby discriminating whether the image as a discrimination target is an image formed of the normal mucosal area, an image formed of the abnormal area, or an image partially including the abnormal area. The threshold used for this discrimination may be calculated using a sample image, stored in advance in the storage unit 32, and called.

Incidentally, the discrimination method based on the histogram of the difference values is not limited to the above-described method, and for example, discrimination may be performed based on statistical values such as a variance, an average, a standard deviation, a median, and a peak half value width of the difference values. In addition, the type of abnormal area may be classified by setting a plurality of threshold values in a stepwise manner.

The image discrimination unit 33b outputs a discrimination result obtained by the discrimination unit 339, and stores the result in the storage unit 32 in association with a display image created by the image processing unit 33a. To be specific, a flag, which indicates that the entire image is the normal mucosal area, the entire image is the abnormal area, or that the image partially includes the abnormal area, is added for the display image. Alternatively, the image formed of the normal mucosal area and the image at least a part of which includes the abnormal area may be stored in storage areas different from each other. Only the image at least a part of which includes the abnormal area may be separately saved for backup.

In addition, when the display image is displayed on the display unit 34, the display may be performed using a different method from the image formed of the normal mucosal area by displaying warning for the image at least a part of which includes the abnormal area. Thereafter, the processing with respect to the data acquired from the imaging unit 2 is completed.

As described above, the subject distance is calculated based the on image data, which depends on the reflection characteristic of the surface of the subject S, the depth to the subject S is calculated based on the distance measurement data which does not depend on the reflection characteristic of the surface of the subject S, and the difference value between the subject distance and the depth is calculated, and thus, it is possible to numerically grasp a change in the surface property of the subject S according to the first embodiment. Accordingly, it is possible to accurately perform the discrimination on whether the image includes the specific area without performing complicated processing by comparing the statistical value of the difference value with the threshold value.

Incidentally, an error may occur in the creation processing of the subject distance image and the depth image in some cases when imaging is performed by directing an imaging direction of the imaging unit 2 to be oriented in a direction in which the lumen extends, in the image discrimination system according to the first embodiment. This is because almost no reflection light comes back from the direction in which the lumen extends. Thus, it is preferable to exclude an image obtained by imaging the direction in which the lumen extends, in advance, from the processing target. It is possible to determine whether or not the image is the image obtained by imaging the direction in which the lumen extends using a known technique. As an example, when an area where the luminance value is a predetermined value or less accounts for a predetermined proportion or more of the entire image in the luminance image created by the luminance image creation unit 331, it is possible to determine as the image obtained by imaging the direction in which the lumen extends.

(First Modified Example)

Although the reflectance of the normal mucosa of the living body is used as the reflectance $R_0$ of the subject S, which is used when the irradiation illuminance calculation unit 334 calculates the irradiation illuminance, in the above-described first embodiment, a reflectance at a specific lesion (for example, a portion where bleeding occurs) may be used. In this case, it is possible to discriminate whether or not an image as a processing target includes a specific lesion area in the discrimination unit 339.

(Second Modified Example)

Although the threshold used by the discrimination unit 339 is set to a fixed value in the above-described first embodiment, the threshold value may be adaptively changed based on a histogram of difference values calculated by the difference calculation unit 338. For example, when two peaks appear in the histogram of difference values as illustrated in FIG. 10, a value between the two peaks is set as a threshold value.

Here, when examining a living body by an endoscope, a reflectance at a mucosa of the living body differs strictly depending on individual living bodies. In addition, there is also a case in which influence on illumination light reflected on the mucosa differs depending on a condition such as a wavelength of the illumination light. Thus, it is possible to improve discrimination accuracy with respect to a plurality of images acquired by the one-time endoscopic examination by adaptively setting the threshold value.

(Third Modified Example)

Although the sensor for image plane phase difference AF in which the plurality of imaging pixels 23b and the plurality of distance measurement pixels 23c are arranged on the same light receiving surface 23a is used as the image sensor 23 in the above-described first embodiment, the configuration of the image sensor 23 is not limited thereto. For example, a general imaging sensor, such as a CMOS and a CCD, and a TOF type distance measurement sensor may be used in combination.

(Second Embodiment)

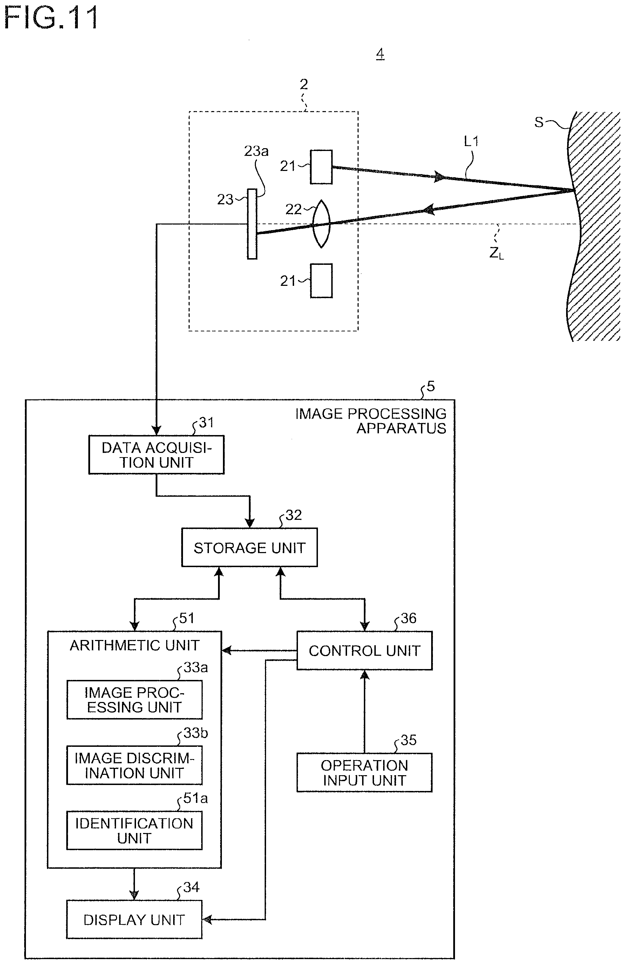

Next, a second embodiment of the present invention will be described. FIG. 11 is a schematic diagram illustrating a configuration example of an image discrimination system according to the second embodiment of the present invention. As illustrated in FIG. 11, an image discrimination system 4 according to the second embodiment includes an image processing apparatus 5 instead of the image processing apparatus 3 illustrated in FIG. 1. Incidentally, the configuration and operation of the imaging unit 2 are the same as those in the first embodiment.

The image processing apparatus 5 includes an arithmetic unit 51 that further includes an identification unit 51a with respect to the arithmetic unit 33 illustrated in FIG. 1. The configuration and operation of each unit of the image processing apparatus 5 other than the arithmetic unit 51 and the operations of the image processing unit 33a and the image discrimination unit 33b included in the arithmetic unit 51 are the same as those in the first embodiment.

The identification unit 51a extracts an abnormal area from an image, which has been discriminated as an image at least a part of which includes the abnormal area by the image discrimination unit 33b, and performs identification processing to identify a type of the extracted abnormal area. It is possible to apply various types of known processing as the extraction processing and identification processing of the abnormal area executed by the identification unit 51a. For example, various types of feature data are calculated by calculating a color feature data of each pixel forming an image with respect to a display image created by the image processing unit 33a, calculating a shape feature data by extracting an edge from the image, or calculating a texture feature data from a Fourier spectrum, and these types of feature data are classified according to identification criteria created in advance.

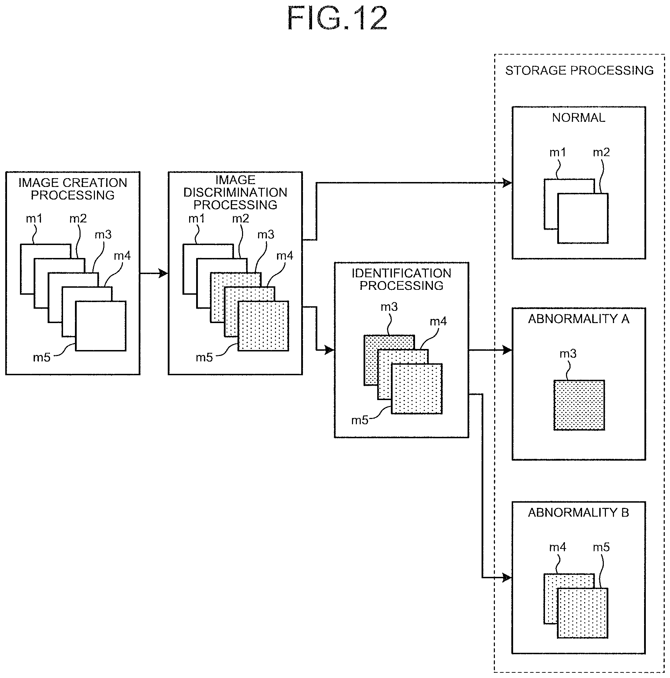

Next, an operation of the arithmetic unit 51 will be described. FIG. 12 is a schematic diagram for describing the operation of the arithmetic unit 51. When the arithmetic unit 51 acquires image data and distance measurement data from the storage unit 32, the image processing unit 33a sequentially creates display images m1 to m5 using the image data.

Subsequently, the image discrimination unit 33b discriminates whether each of the images m1 to m5 is an image formed of a normal mucosal area or the image at least a part of which includes the abnormal area. Details of this image discrimination processing are the same as those in the first embodiment.

For example, when each of the images m1 and m2 is discriminated as the image formed of the normal mucosal area through the above-described image discrimination processing, the arithmetic unit 51 adds a flag indicating the image formed of the normal mucous area to the images m1 and m2 and stores the images in a predetermined storage area of the storage unit 32.

In addition, when each of the images m3 to m5 is discriminated as the image at least a part of which includes the abnormal area through the above-described image discrimination processing, the identification unit 51a extracts the abnormal area from each of these images m3 to m5 and executes the identification processing to identify the type of the extracted abnormal area.

When it is discriminated that a type (for example, abnormality A) of the abnormal area included in the image m3 is different from a type (for example, abnormality B) of the abnormal areas included in the images m4 and m5 through this identification process, the arithmetic unit 51 adds flags indicating the identified types of the abnormal areas to these images m3 to m5, and stores the images in a predetermined storage area corresponding to the respective types.

As described above, since the advanced image processing to extract the abnormal area and identify the type of the abnormal area is performed only for the image discriminated as the image including the abnormal area, it is possible to efficiently obtain an identification result for a necessary image while suppressing a load on the image processing apparatus according to the second embodiment.

(Third Embodiment)

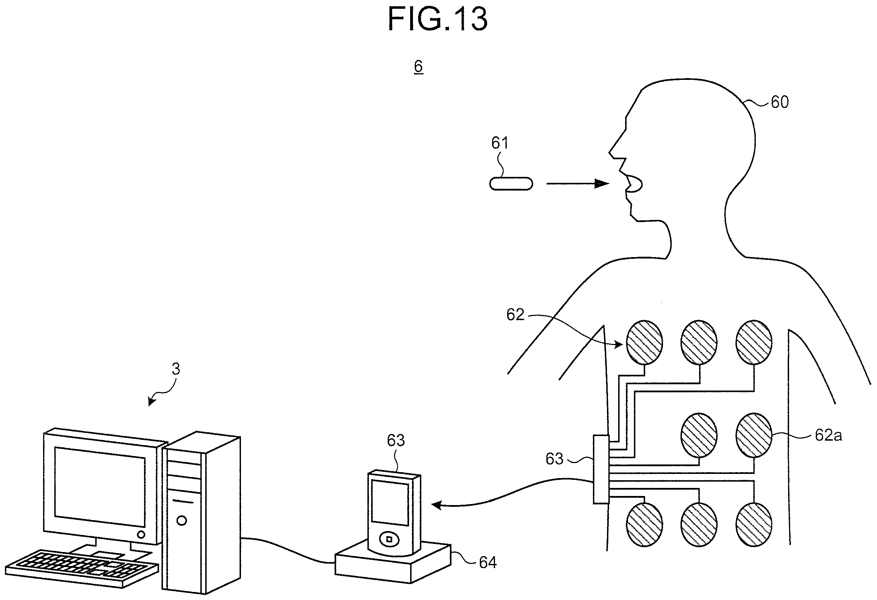

Next, a third embodiment of the present invention will be described. FIG. 13 is a schematic diagram illustrating a configuration of an endoscopic system according to the third embodiment of the present invention. As illustrated in FIG. 13, an endoscopic system 6 according to the third embodiment includes a capsule endoscope 61 that is introduced into a subject 60, such as a patient, and performs imaging to generate and wirelessly transmit an image signal, a receiving device 63 that receives the image signal wirelessly transmitted from the capsule endoscope 61 via a receiving antenna unit 62 mounted to the subject 60, and the image processing apparatus 3. The image processing apparatus 3 has the same configuration and operation as those of the first embodiment (see FIG. 1), acquires image data from the receiving device 63, performs predetermined image processing on the image data, and displays an image inside the subject 60. Alternatively, the image processing apparatus 5 according to the second embodiment may be applied instead of the image processing apparatus 3.

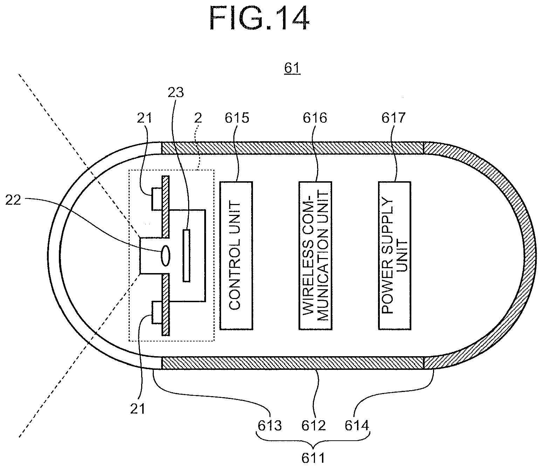

FIG. 14 is a schematic diagram illustrating a configuration example of the capsule endoscope 61. The capsule endoscope 61 is introduced into the subject 60 by ingestion or the like, and then, moves inside a digestive tract and finally is discharged to the outside of the subject 60. In the meantime, the capsule endoscope 61 captures the inside of the subject 60, sequentially generates image signals, and wirelessly transmits the generated image signals while moving inside an internal organ (digestive tract) by peristaltic motion.

As illustrated in FIG. 14, the capsule endoscope 61 includes a capsule-shaped casing 611 that accommodates the imaging unit 2 including the illumination unit 21, the condensing optical system 22, and the image sensor 23. The capsule-shaped casing 611 is an outer casing which is formed in a size that can be easily introduced into the organ of the subject 60. In addition, a control unit 615 that controls each configuration portion of the capsule endoscope 61, a wireless communication unit 616 that wirelessly transmits the signal processed by the control unit 615 to the outside of the capsule endoscope 61, and a power supply unit 617 that supplies power to each configuration unit of the capsule endoscope 61 are provided inside the capsule-shaped casing 611.

The capsule-shaped casing 611 is configured of a cylindrical casing 612 and dome-shaped casings 613 and 614, and is realized by closing opening ends on both sides of the cylindrical casing 612 with the dome-shaped casings 613 and 614. The cylindrical casing 612 and the dome-shaped casing 614 are colored casings that are substantially opaque to visible light. On the other hand, the dome-shaped casing 613 is an optical member which has a dome shape and is transparent to light of a predetermined wavelength band such as visible light. The capsule-shaped casing 611 configured in this manner liquid-tightly encloses the imaging unit 2, the control unit 615, the wireless communication unit 616, and the power supply unit 617.

The control unit 615 controls the operation of each configuration unit inside the capsule endoscope 61 and controls input and output of signals among these configuration units. In detail, the control unit 615 controls an imaging frame rate of the image sensor 23 included in the imaging unit 2, and causes the illumination unit 21 to emit light in synchronization with the imaging frame rate. In addition, the control unit 615 performs predetermined signal processing on the image signal output from the image sensor 23, and wirelessly transmits the image signal through the wireless communication unit 616.

The wireless communication unit 616 acquires the image signal from the control unit 615, performs modulation processing and the like on the image signal to generate a wireless signal, and transmits the wireless signal to the receiving device 63.

The power supply unit 617 is a power storage unit, such as a button battery and a capacitor, and supplies power to the respective configuration units (the imaging unit 2, the control unit 615, and the wireless communication unit 616) of the capsule endoscope 61.

Referring again to FIG. 13, the receiving antenna unit 62 has a plurality of (eight in FIG. 13) receiving antennas 62a. Each of the receiving antennas 62a is realized using, for example, a loop antenna, and is arranged at a predetermined position on an external surface of the body of the subject 60 (for example, a position corresponding to each organ inside the subject 60 which is a passage area of the capsule endoscope 61).

The receiving device 63 receives the image signal wirelessly transmitted from the capsule endoscope 61 via these receiving antennas 62a, performs predetermined processing on the received image signal, and then, stores the image signal and related information thereof in a built-in memory. The receiving device 63 may be provided with a display unit, which displays a reception state of the image signal wirelessly transmitted from the capsule endoscope 61, and an input unit such as an operation button to operate the receiving device 63. The image signal stored in the receiving device 63 is transferred to the image processing apparatus 3 by setting the receiving device 63 to a cradle 64 connected to the image processing apparatus 3.

(Fourth Embodiment)

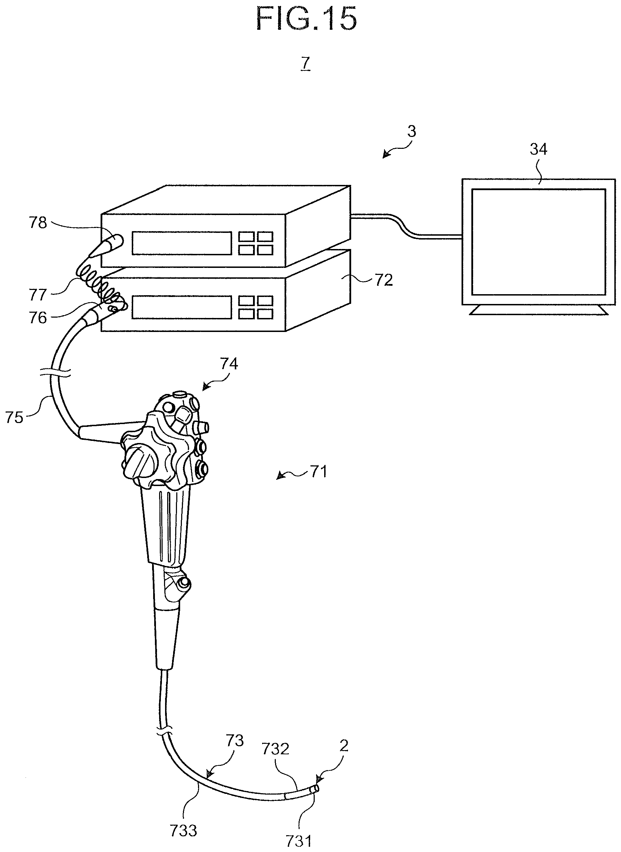

Next, a fourth embodiment of the present invention will be described. FIG. 15 is a schematic diagram illustrating a configuration of an endoscopic system according to the fourth embodiment of the present invention. As illustrated in FIG. 15, an endoscopic system 7 according to the fourth embodiment includes an endoscope 71 that is inserted into a body of a subject and performs imaging to generate and output an image, a light source device 72 that generates illumination light to be emitted from a distal end of the endoscope 71, and the image processing apparatus 3. The image processing apparatus 3 have the same configuration and operation as those of the first embodiment (see FIG. 1), acquires image data generated by the endoscope 71, performs various types of image processing on the image data, and displays an image inside the subject on the display unit 34. Alternatively, the image processing apparatus 5 according to the second embodiment may be applied instead of the image processing apparatus 3.

The endoscope 71 includes an insertion portion 73 that has flexibility and is formed in an elongated shape, an operating unit 74 that is connected to a proximal end side of the insertion portion 73 and receives input of various operation signals, and a universal cord 75 that extends from the operating unit 74 in a direction different from an extending direction of the insertion portion 73 and includes various built-in cables which are connected to the image processing apparatus 3 and the light source device 72.

The insertion portion 73 has a distal end portion 731, a bendable bending portion 732 configured using a plurality of bending pieces, an elongated flexible needle tube 733 which has flexibility and is connected to a proximal end side of the bending portion 732. The imaging unit 2 (see FIG. 1), which includes the illumination unit 21 that irradiates the inside of the subject with the illumination light generated by the light source device 72, the condensing optical system 22 that condenses the illumination light reflected inside the subject, and the image sensor 23, is provided at the distal end portion 731 of the insertion portion 73.

A cable assembly in which a plurality of signal lines to transmit and receive an electric signal to and from the image processing apparatus 3 is bundled and a light guide to transmit light are connected between the operating unit 74 and the distal end portion 731. The plurality of signal lines includes a signal line to transmit an image signal output from an imaging element to the image processing apparatus 3, a signal line to transmit a control signal output from the image processing apparatus 3 to the imaging element, and the like.

The operating unit 74 is provided with a treatment tool insertion portion into which treatment tools, such as a bending knob to bend the bending portion 732 in a vertical direction and a lateral direction, a biopsy needle, biopsy forceps, a laser scalpel, and an examination probe, are inserted, and a plurality of switches which is configured to input an operation indicating signal to peripheral devices such as the image processing apparatus 3 and the light source device 72.

The universal cord 75 at least incorporates the light guide and the cable assembly. In addition, a connector portion 76 detachably attached to the light source device 72 and an electrical connector portion 78, which is electrically connected to the connector portion 76 via a coil-shaped coil cable 77 and detachably attached to the image processing apparatus 3, are provided at an end portion on a side of the universal cord 75 which is different from the side connected to the operating unit 74. The image signal output from the imaging element is input to the image processing apparatus 3 via the coil cable 77 and the electrical connector portion 78.

The above-described first to fourth embodiments of the present invention are only examples for implementation of the present invention, and the present invention is not limited thereto. In addition, the present invention allows various inventions to be formed by appropriately combining a plurality of components disclosed in the above-described first to fourth embodiments. The present invention can be modified in various manners in accordance with specifications. In addition, it is obvious that other various embodiments can be implemented within a scope of the present invention, from the above description. Reference Signs List

| | | |
|---|---|---|
| 1, 4 | IMAGE DISCRIMINATION SYSTEM | |
| 2 | IMAGING UNIT | |
| 3, 5 | IMAGE PROCESSING APPARATUS | |
| 6, 7 | ENDOSCOPIC SYSTEM | |
| 21 | ILLUMINATION UNIT | |
| 22 | CONDENSING OPTICAL SYSTEM | |
| 23 | IMAGE SENSOR | |
| 23a | LIGHT RECEIVING SURFACE | |
| 23b | IMAGING PIXEL | |
| 23c | DISTANCE MEASUREMENT PIXEL | |
| 23d | SIGNAL PROCESSING CIRCUIT | |
| 31 | DATA ACQUISITION UNIT | |
| 32 | STORAGE UNIT | |
| 33, 51 | ARITHMETIC UNIT | |
| 33a | IMAGE PROCESSOR | |
| 33b | IMAGE DISCRIMINATION UNIT | |
| 34 | DISPLAY UNIT | |
| 35 | OPERATION INPUT UNIT | |
| 36 | CONTROL UNIT | |
| 51a | IDENTIFICATION UNIT | |
| 60 | SUBJECT | |
| 61 | CAPSULE ENDOSCOPE | |
| 62 | RECEIVING ANTENNA UNIT | |
| 62a | RECEIVING ANTENNA | |
| 63 | RECEIVING DEVICE | |
| 64 | CRADLE | |
| 71 | ENDOSCOPE | |
| 72 | LIGHT SOURCE DEVICE | |
| 73 | INSERTION PORTION | |
| 74 | OPERATING UNIT | |
| 75 | UNIVERSAL CORD | |
| 76 | CONNECTOR UNIT | |
| 77 | COIL CABLE | |
| 78 | ELECTRICAL CONNECTOR PORTION | |
| 331 | LUMINANCE IMAGE CREATION UNIT | |
| 332 | IMAGE PLANE ILLUMINANCE CALCULATION UNIT | |
| 333 | OBJECT PLANE LUMINANCE CALCULATION UNIT | |
| 334 | IRRADIATION ILLUMINANCE CALCULATION UNIT | |
| 335 | IRRADIATION DISTANCE CALCULATION UNIT | |
| 336 | SUBJECT DISTANCE CALCULATION UNIT | |
| 337 | DEPTH IMAGE CREATION UNIT | |
| 338 | DIFFERENCE CALCULATION UNIT | |
| 339 | DISCRIMINATION UNIT | |
| 611 | CAPSULE-SHAPED CASING | |
| 612 | TUBULAR CASING | |
| 613, 614 | DOME-SHAPED CASING | |
| 615 | CONTROL UNIT | |
| 616 | WIRELESS COMMUNICATION UNIT | |
| 617 | POWER SUPPLY UNIT | |
| 731 | DISTAL END PORTION | |
| 732 | BENDING PORTION | |
| 733 | FLEXIBLE NEEDLE TUBE | |

**Claims**

1. An image processing apparatus that performs image processing based on image data and distance measurement data output from an imaging unit including an illumination unit configured to generate illumination light for irradiation of a subject, a condensing optical system configured to condense the illumination light reflected by the subject, and an image sensor configured to receive the illumination light condensed by the condensing optical system and to output the image data representing an image of the subject and output the distance measurement data representing a distance to the subject based on the illumination light, the image processing apparatus comprising:

   a depth calculation unit configured to calculate a depth from the imaging unit to the subject based on the distance measurement data;
   a subject distance calculation unit configured to calculate a subject distance between the imaging unit and the subject based on the image data;
   a difference calculation unit configured to calculate a difference between the depth calculated by the depth calculation unit and the subject distance calculated by the subject distance calculation unit; and
   a discrimination unit configured to discriminate whether or not an area where a surface of the subject is in a specific state is included in an image in which the subject is imaged based on the difference calculated by the difference calculation unit.

2. The image processing apparatus according to claim 1, wherein
   the depth calculation unit calculates depths to a plurality of points on the subject imaged on a plurality of pixels forming the image,
   the subject distance calculation unit calculates at least subject distances to a plurality of points on the subject for which the depth calculation unit calculates the depth, among the plurality of pixels forming the image,
   the difference calculation unit calculates difference values between distances to a common point, between the plurality of depths calculated by the depth calculation unit and the plurality of subject distances calculated by the subject distance calculation unit, and
   the discrimination unit performs discrimination based on a statistical value of the plurality of difference values calculated by the difference calculation unit.

3. The image processing apparatus according to claim 2, wherein
   the discrimination unit performs the discrimination by comparing a difference value having a peak frequency among the plurality of difference values with a threshold value.

4. The image processing apparatus according to claim 1, further comprising
   an identification unit configured to perform identification processing to identify a type of the specific state with respect to an image that is discriminated to include the area in the specific state by the discrimination unit.

5. The image processing apparatus according to claim 4, wherein
   the subject is a mucosa of a living body, and
   the specific state is an abnormal state in which the mucosa is changed from a normal state.

6. An image discrimination system comprising:

   the image processing apparatus according to claim 1; and
   the imaging unit.

7. The image discrimination system according to claim 6, wherein the image sensor comprises:

   a plurality of imaging pixels that receives the illumination light and outputs an electric signal representing the image of the subject;
   a plurality of distance measurement pixels that receives the illumination light and outputs an electric signal containing information on the distance to the subject; and
   a signal processing circuit that outputs the image data based on the electrical signal output from each of the plurality of imaging pixels and outputs the distance measurement data based on the electric signal output from each of the plurality of distance measurement pixels.

8. An endoscopic system comprising:

the image processing apparatus according to claim 1; and
a capsule endoscope accommodating the imaging unit in a capsule-shaped casing.

9. An endoscopic system comprising:

the image processing apparatus according to claim 1; and
a video scope provided with the imaging unit at a distal end portion of an insertion portion to be inserted into the subject.

# FIG.1

# FIG.2

SIGNAL PROC-
ESSING CIRCUIT

~23d

TO IMAGE PROCESSING
APPARATUS

# FIG.3

IMAGE DATA

DISTANCE MEASUREMENT DATA

33b

IMAGE DISCRIMINATION UNIT

331

LUMINANCE IMAGE CREATION UNIT

332

IMAGE PLANE IL-LUMINANCE CAL-CULATION UNIT

333

OBJECT PLANE LUMINANCE CAL-CULATION UNIT

334

IRRADIATION IL-LUMINANCE CAL-CULATION UNIT

335

IRRADIATION DISTANCE CALCULATION UNIT

336

SUBJECT DISTANCE CALCULATION UNIT

337

DEPTH IMAGE CREATION UNIT

338

DIFFERENCE CALCULATION UNIT

339

DISCRIMINATION UNIT

# FIG.4

# FIG.5

$d_s(x, y)$ M1

$d_s'(x, y)$ M2

# FIG.6

FREQUENCY

DIFFERENCE VALUE

Th

# FIG.7

$d_s(x, y)$

M3

$d_s'(x, y)$

M4

# FIG.8

FREQUENCY

DIFFERENCE
VALUE

Th

# FIG.9

$d_s(x, y)$     M5     b1     $d_s'(x, y)$     M6     b2

a1          a2

# FIG.10

FREQUENCY

DIFFERENCE
VALUE

Th

# FIG.11

# FIG.12

STORAGE PROCESSING

IMAGE CREATION
PROCESSING

m1  m2
m3
m4
m5

IMAGE
DISCRIMINATION
PROCESSING

m1  m2
m3
m4
m5

IDENTIFICATION
PROCESSING

m3
m4
m5

NORMAL

m1  m2

ABNORMALITY A

m3

ABNORMALITY B

m4  m5

# FIG.13

# FIG.14

61

23

21   2   615      616      617

22

| CONTROL UNIT | WIRELESS COM-MUNICATION UNIT | POWER SUPPLY UNIT |

21

613   612   614

611

# FIG.15

7

## INTERNATIONAL SEARCH REPORT

| International application No. |
| --- |
| PCT/JP2016/054452 |

**A. CLASSIFICATION OF SUBJECT MATTER**
*A61B1/04*(2006.01)i, *A61B1/00*(2006.01)i, *G02B23/24*(2006.01)i, *H04N7/18* (2006.01)i

According to International Patent Classification (IPC) or to both national classification and IPC

**B. FIELDS SEARCHED**

Minimum documentation searched (classification system followed by classification symbols)
A61B1/00-1/32

Documentation searched other than minimum documentation to the extent that such documents are included in the fields searched
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2009/154125 A1 (Olympus Corp.), 23 December 2009 (23.12.2009), paragraphs [0013] to [0060] & US 2011/0085717 A1 paragraphs [0036] to [0084] & JP 2009-297450 A    & EP 2305091 A1 & CN 102065744 A | 1-9 |
| A | JP 2015-119277 A (Olympus Imaging Corp.), 25 June 2015 (25.06.2015), paragraphs [0014] to [0108] & CN 104717422 A | 1-9 |
| A | JP 2002-065585 A (Fuji Photo Film Co., Ltd.), 05 March 2002 (05.03.2002), paragraphs [0030] to [0096] (Family: none) | 1-9 |

☒ Further documents are listed in the continuation of Box C. ☐ See patent family annex.

| | |
| --- | --- |
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>10 May 2016 (10.05.16) | Date of mailing of the international search report<br>17 May 2016 (17.05.16) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
|---|
| PCT/JP2016/054452 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-204991 A  (Funai Electric Co., Ltd.),<br>10 September 2009 (10.09.2009),<br>paragraphs [0020] to [0048]<br>(Family: none) | 1-9 |

Form PCT/ISA/210 (continuation of second sheet) (January 2015)

**EP 3 318 176 A1**

**REFERENCES CITED IN THE DESCRIPTION**

**Patent documents cited in the description**

* JP 2009297450 A